(19) Europäisches Patentamt
European Patent Office
Office européen des brevets

(11) Veröffentlichungsnummer: **0 148 742**
**B1**

(12) **EUROPÄISCHE PATENTSCHRIFT**

(45) Veröffentlichungstag der Patentschrift: **02.11.88**

(51) Int. Cl.⁴: **C 07 D 471/04**, A 61 K 31/44 // (C07D471/04, 235:00, 221:00)

(21) Anmeldenummer: **84890212.8**

(22) Anmeldetag: **09.11.84**

(54) **2-(2-Thienyl)-imidazo 4,5-b pyridin-derivate und pharmazeutisch verträgliche Säureadditionssalze hiervon sowie Verfahren zu deren Herstellung.**

(30) Priorität: **14.11.83 AT 3999/83**

(43) Veröffentlichungstag der Anmeldung: **17.07.85 Patentblatt 85/29**

(45) Bekanntmachung des Hinweises auf die Patenterteilung: **02.11.88 Patentblatt 88/44**

(84) Benannte Vertragsstaaten: **BE CH DE FR GB IT LI LU NL SE**

(56) Entgegenhaltungen:
**EP-A-0 072 926**
**DE-A-2 305 339**
**DE-A-3 225 386**
**US-A-4 336 257**

**CHEMICAL ABSTRACTS, Band 62, Nr. 4, 15. Februar 1965, Columbus, Ohio, USA; D.L. GARMAISE et al. "Preparation of 2-arylimidazo-(4,5-b)pyridines", Spalte 4022b**
**Medicin of Chemistry, 3. Auflage Teil 1; Alfred Burger, Wiley Interscience (1970), S. 77**
**Medicin of Chemistry, 4. Auflage, Teil 1 (1979) S. 4,5**
**Arch. Pharm. (Weinheim) 318, 243-249 (1985)**
**Arch. Pharm. (Weinheim) 318, 210-213 (1985)**
**Arch. Pharm. (Weinheim) 318, 70-78 (1985)**
**Arch. Pharm. (Weinheim) 318, 48-59 (1985)**
**Arch. Pharm. (Weinheim) 314, Heft 6, 564-567**
**Arch. Pharm. (Weinheim) 318, 40-48 (1985)**

(73) Patentinhaber: **Laevosan- Gesellschaft m.b.H., Estermannstrasse 17, A-4020 Linz (AT)**

(72) Erfinder: **Binder, Dieter, Prof. Dr., Sieveringerstrasse 207, A-1190 Wien (AT)**
Erfinder: **Rovenszky, Franz, Dipl.- Ing., Lagerhausstrasse 5/8, A-2460 Bruck/Leitha (AT)**

(74) Vertreter: **Pawloy, Heinrich, Dr., Patentanwälte Dr. Heinrich Pawloy Dipl.- Ing. Helmut Sonn Dipl.- Ing. Arnulf Weinzinger Riemergasse 14, A-1010 Wien (AT)**

(56) Entgegenhaltungen: (Fortsetzung)
**Arch. Pharm. (Weinheim) 313, Heft 10, 883-890**
**Arch. Pharm. (Weinheim) 313, Heft 7, 587-602**
**Arch. Pharm. (Weinheim) 312 Heft 2, 169-174**

**Die Akte enthält technische Angaben, die nach dem Eingang der Anmeldung eingereicht wurden und die nicht in dieser Patentschrift enthalten sind.**

**EP 0 148 742 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue therapeutisch wertvolle Derivate von 2-(2-Thienyl)-imidazo[4,5-b]pyridinen, ein Verfahren zu deren Herstellung und sie enthaltende pharmazeutische Zusammensetzungen.

Insbesondere betrifft die Erfindung Verbindungen der allgemeinen Formel (I)

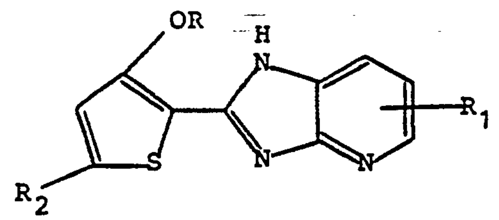

(I),

worin R Methyl oder Äthyl, $R_1$ Wasserstoff oder Methyl und $R_2$ Methylthio, Methylsulfinyl oder Methoxy bedeuten, und ihre pharmazeutisch verträglichen Säureadditionssalze.

Die Verbindungen der obigen allgemeinen Formel (I) besitzen wertvolle pharmakologische Eigenschaften, insbesondere eine positiv inotrope Wirksamkeit auf das Herz.

Das erfindungsgemäße Verfahren ist dadurch gekennzeichnet, daß man

a) eine Verbindung der Formel

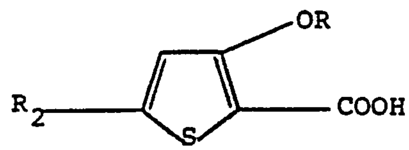

(II),

worin R die obige Bedeutung besitzt und $R_2$ Methylthio oder Methoxy bedeutet,

mit einer Verbindung der Formel

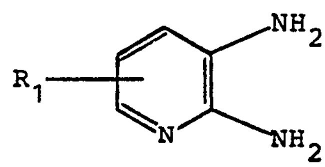

(III),

worin $R_1$ die obige Bedeutung besitzt, in Gegenwart von Phosphoroxychlorid oder Thionylchlorid umsetzt, und

b) erforderlichenfalls die so erhaltene Verbindung der allgemeinen Formel (I), in der $R_2$ Methylthio bedeutet, mit organischen Persäuren oder Wasserstoffperoxid in eine Verbindung der allgemeinen Formel (I) umwandelt, in der $R_2$ Methylsulfinyl bedeutet.

Die Umsetzung nach Verfahrensschritt a) wird mit Phosphoroxychlorid oder Thionylchlorid als wasserentziehendes Kondensationsmittel durchgeführt, wobei diese am besten gleich als Lösungsmittel verwendet werden. Die bevorzugte Reaktionstemperatur ist die Rückflußtemperatur des jeweiligen Lösungsmittels.

Die Herstellung der Sulfinylverbindungen der allgemeinen Formel (I), gemäß Verfahrensschritt b), ausgehend von den erhaltenen Methylmercaptoverbindungen der allgemeinen Formel (I) kann mit berechneten Mengen organischer Persäuren, z. B. Peressigsäure oder m-Chlorperbenzoesäure, in einem reaktionsinerten Lösungsmittel, z. B. Methylenchlorid oder Chloroform, bei Temperaturen um 0°C, oder mit berechneten Mengen von 30 %-igem Wasserstoffperoxid in Eisessig bei Raumtemperatur durchgeführt werden.

Die Verbindungen der allgemeinen Formel (I) haben stark basische Eigenschaften. Man kann sie daher auch leicht in kristalline, pharmazeutisch verträgliche Säureadditionssalze überführen, die sich wie z. B. die Hydrochloride gut durch Umkristallisieren reinigen lassen. Dazu löst man die rohe Base in einem geeigneten Lösungsmittel, z. B. in einem niederen Alkohol, gibt eine äquivalente Menge Protonensäure dazu, dampft im Vakuum das Lösungsmittel ab und kristallisiert den Rückstand aus Methanol oder Äthanol, gegebenenfalls unter Zusatz von Äther, um. Geeignete Beispiele für derartige pharmazeutisch verträgliche Salze sind neben dem Salz der Salzsäure, das der Schwefelsäure, der Salpetersäure, der Phosphorsäure, von Sulfonsäuren, der Benzoesäure, der Maleinsäure, der Weinsäure und der Zitronensäure.

Die erfindungsgemäß erhältlichen Säureadditionssalze haben eine ebenso große positiv inotrope Wirkung wie die entsprechenden freien Basen der Formel (I).

Die Verbindungen der Formel (III) sind literaturbekannt. Die Verbindungen der Formel (II) kann man, ausgehend von den literaturbekannten Verbindungen der Formel (IV), auf folgendem, dem Fachmann geläufigem Syntheseweg herstellen:

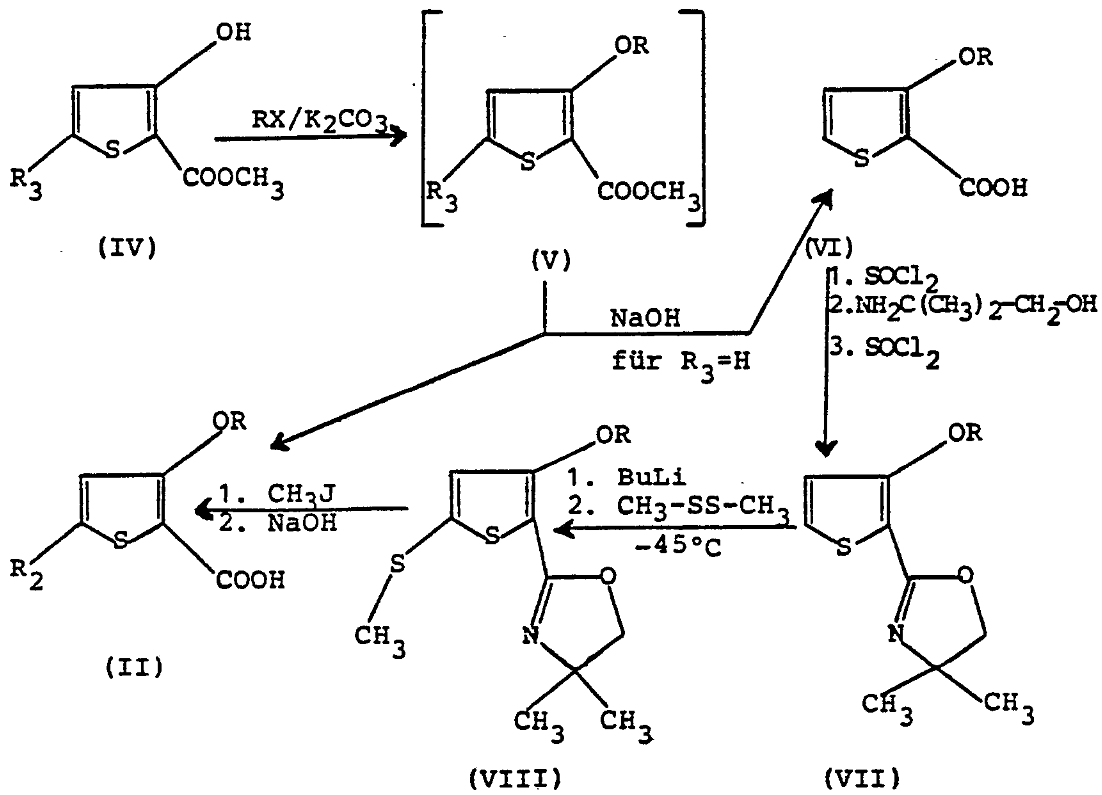


(IV) (V) (VI) (II) (VIII) (VII)

R $CH_3$ oder $C_2H_5$
$R_2$ Methylthio oder Methoxy
$R_3$ Wasserstoff oder Methoxy
X Br, J oder O-$SO_2$-OR

In den DE-B-2 305 379 und EP-A-0 072 926 werden Verbindungen geoffenbart, die den erfindungsgemäßen Verbindungen strukturell nahe liegen, jedoch anstelle des Thiophenringes einen Benzolring aufweisen und ebenfalls Positiv inotrop wirksam sind.

Die folgenden Beispiele sollen die vorliegende Erfindung erläutern:

**Beispiel 1**: 2-(3Methoxy-5-methylthio-2-thienyl)-1H-imidaso[4,5-b]pyridin (Formel (I); R = $CH_3$, $R_1$ = H und $R_2$ = $SCH_3$)

6,83 g (33,4 m-Mol) 3-Methoxy-5-methylthio-2-thiophencarbonsäure (Formel (II); R = $CH_3$, $R_2$ = $SCH_3$) werden zusammen mit 3,65 g (33,4 mMol) 2,3-Diaminopyridin (Formel (III); $R_1$ = H) in 80 ml Phosphoroxychlorid 3 h am Rückfluß erhitzt Dann wird das erhaltene Reaktionsgemisch portionsweise in ca. 1,5 l kaltes Wasser eingetragen, gut gerührt, über Hyflo filtriert, mit Kaliumcarbonat und etwas Ammoniak alkalisch gemacht und mehrmals mit Methylenchlorid extrahiert. Die vereinigten organischen Phasen werden über Natriumsulfat getrocknet und eingedampft. Der dunkle Rückstand wird in ca. 300 ml Methanol suspendiert, mit überschüssiger methanolischer Salzsäure versetzt, zum Sieden erhitzt, Aktivkohle zugegeben, filtriert, die Lösung auf ca. 100 ml eingeengt und abkühlen gelassen. Das ausfallende gelbe Hydrochlorid von (I) wird abgesaugt und mit wenig Methanol gewaschen. Zur Herstellung der freien Base werden diese Kristalle in 28 ml Methanol suspendiert, 1,2 g 25 %-ige wässerige Ammoniaklösung zugetropft, 80 ml Wasser zugegeben und die ausfallenden gelblichen Kristalle abgesaugt, mit Wasser gewaschen und getrocknet. Ausbeute 4,24 g (46 %), Fp. (Äthanol): 224° C (Zers.).

Das Ausgangsmaterial kann wie folgt hergestellt werden:

3-Methoxy-2-thiophencarbonsäure (VI; R = $CH_3$)

48,5 g (0,31 mMol) 3-Hydroxy-2-thiophencarbonsäuremethylester (IV; $R_3$ = H) werden in 400 ml Aceton gelöst, 84,7 g (0,61 Mol) Kaliumcarbonat und 77,3 g (0,61 Mol) Dimethylsulfat werden zugegeben und das Gemisch wird unter Rühren 1 h am Rückfluß erhitzt. Dann wird die Salzmasse abgesaugt, mehrmals mit Aceton gewaschen und das vereinigte Filtrat im Vakuum eingeengt; 750 ml wässerige 2n Natronlauge werden zugegeben und es wird kurz aufgekocht. Die Reaktionslösung wird abgekühlt, mit Äther gewaschen und mit konz. HCl angesäuert. Die ausfallenden farblosen Kristalle werden abgesaugt und aus Äthanol umkristallisiert. Ausbeute 43,1 g (89 %), Fp. : 184 - 185° C.

3,4-Dihydro-4,4-dimethyl-2-(3-methoxy-2-thienyl)-oxazol (VII; R = $CH_3$)

50,2 g (0,32 Mol) 3-Methoxy-2-thiophencarbonsäure werden in 300 ml Thionylchlorid 2 h am Rückfluß erhitzt, die Reaktionslösung im Vakuum eingedampft, der kristalline Rückstand (56 g) in 250 ml Methylenchlorid aufgenommen und bei 0° C unter Rühren zu einer Lösung von 57 g (0,64 Mol) 2-Amino-2-methyl-1-propanol in 200 ml Methylenchlorid während 1/2 h zugetropft. Nach 12 h Rühren bei Raumtemperatur wird im Vakuum eingeengt, abgekühlt, der ausgefallene Niederschlag abgesaugt, mit Wasser gut gewaschen, getrocknet, unter Kühlen langsam mit 135 ml Thionylchlorid versetzt und 12 h bei Raumtemperatur gerührt. Dann wird im Vakuum eingedampft, der Rückstand in Wasser gelöst, mit Äther extrahiert, die wässerige Phase mit 2n Natronlauge alkalisch gemacht und mehrmals mit Methylenchlorid extrahiert. Die vereinigten Methylenchloridphasen werden mit Natriumsulfat getrocknet und im Vakuum eingedampft. Ausbeute 57,0 g (84 %), Fp. (Petroläther) 97 - 98° C.

3,4-Dihydro-4,4-dimethyl-2-(3-methoxy-5-methylthio-2-thienyl)-oxazol (VIII; R = $CH_3$)

27 g (0,128 Mol) 3,4-Dihydro-4,4-dimethyl-2-(3-methoxy-2-thienyl)-oxazol werden in 400 ml abs. Tetrahydrofuran gelöst, die Lösung auf -45° C abgekühlt und 88 ml (0,141 Mol) einer 1,6 molaren Lösung von Butyllithium in n-Hexan so zugetropft, daß die Temperatur im Reaktionsgemisch -45° C nicht überschreitet. Nach beendeter Zugabe wird noch 1 1/2 h bei -45° C gerührt, eine Lösung von 13,9 g (0,147 Mol) Dimethyldisulfid in 70 ml abs. Tetrahydrofuran wird zugegeben und noch 30 min bei dieser Temperatur gerührt. Dann wird auf -20° C erwärmt, auf Wasser gegossen und mit Äther mehrmals extrahiert. Die vereinigten Ätherphasen werden mit Natriumsulfat getrocknet, mit Aktivkohle entfärbt, im Vakuum eingedampft und der kristalline Rückstand aus Petroläther umkristallisiert. Ausbeute 24,7 g (75 %), Fp. 56 - 58° C.

3-Methoxy-5-methylthio-2-thiophencarbosäure (II; R = $CH_3$, $R_2$ = $SCH_3$)

24,2 g (0,094 Mol) 3,4-Dihydro-4,4-dimethyl-2-(3-methoxy-5-methylthio-2-thienyl)oxazol werden mit 250 ml Methyljodid versetzt und 12 h bei Raumtemperatur gerührt. Dann wird das überschüssige Methyljodid abdestilliert, der kristalline Rückstand mit 350 ml 1n Natronlauge versetzt, weitere 12 h bei Raumtemperatur gerührt, die Reaktionsmischung mit Wasser verdünnt, bis sich der gelbe Niederschlag löst, mit Äther extrahiert, die wässerige Phase mit konz. Salzsäure angesäuert und die ausfallenden farblosen Kristalle

abgesaugt, mit Wasser gewaschen und getrocknet. Ausbeute 15,8 g (82 %) Fp. ($CCl_4$): 126 - 128°C (Zers.).

**Beispiel 2**: 2-(3-Methoxy-5-methylsulfinyl-2-thienyl)-1H-imidazo[4,5-b]pyridin (1; R = $CH_3$, $R_1$ = H und $R_2$ = SO-$CH_3$)

3,80 g (13,7 mMol) des nach Beispiel 1 erhaltenen 2-(3-Methoxy-5-methylthio-2-thienyl)-1H-imidazo[4,5-b]pyridins werden in 80 ml Eisessig gelöst, mit 1,63 g (14,4 mMol) 30 %-iger wässeriger Wasserstoffperoxidlösung versetzt und 30 h bei Raumtemperatur gerührt. Dann wird im Vakuum eingeengt, der Rückstand in wenig Wasser gelöst, mit gesättigter Natriumhydrogencarbonatlösung neutralisiert und die ausfallenden Kristalle werden abgesaugt und getrocknet. Ausbeute 3,95 g (98 %), Fp. (Äthanol) 233 - 235°C (Zers.).

**Beispiel 3**: 2-(3,5-Dimethoxy-2-thienyl)-1H-imidazo-[4,5-b]pyridin (Formel (I); R = $CH_3$, $R_1$ = H und $R_2$ = $OCH_3$)

Bei Umsetzung von 3,5-Dimethoxy-2-thiophencarbonsäure (Formel II; R = $CH_3$, $R_2$ = O-$CH_3$) auf analoge Weise, wie in Beispiel 1 beschrieben, wird die Titelverbindung in 29 %-iger Ausbeute in Form gelblicher Kristalle erhalten. Wenn gewünscht, kann aus Äthanol umkristallisiert werden, Fp. 93 - 96°C.

Das Ausgangsmaterial kann wie folgt hergestellt werden:

3,5-Dimethoxy-2-thiophencarbonsäuremethylester

8,0 g (0,0425 Mol) 3-Hydroxy-5-methoxy-2-thiophencarbonsäuremethylester, 6,5 g (0,0468 Mol) Kaliumcarbonat und 5,9 g (0,0468 Mol) Dimethylsulfat werden in 100 ml Aceton 1 h am Rückfluß erhitzt. Anschließend wird das Reaktionsgemisch eingedampft, der Rückstand zwischen Wasser und Diäthyläther verteilt und die wässerige Phase dreimal mit insgesamt 200 ml Diäthyläther extrahiert. Die organische Phase wird über Natriumsulfat/Aktivkohle getrocknet und eingedampft. Ausbeute 8,62 g gelbliche Kristalle (99,8 % d. Th.), Fp. 62 - 64°C (Petroläther).

3,5-Dimethoxy-2-thiophencarbonsäure

10,0 g (0,0492 Mol) 3,5-Dimethoxy-2-thiophencarbonsäuremethylester werden in 80 ml 2n NaOH 1 h am Rückfluß erhitzt. Anschließend wird auf 0°C gekühlt, mit wenig kaltem Wasser verdünnt und bei 0°C tropfenweise mit konz.HCl auf pH 1,5 angesäuert. Es wird kurz gerührt, die ausgefallenen Kristalle werden abgesaugt, mit kaltem Wasser und wenig kaltem Methanol gewaschen und kurz trockengesaugt. Die Kristalle werden bei 20°C/2 mbar getrocknet. Ausbeute 4,2 g farblose Kristalle (45,4 % d.Th.), Fp. 135°C (Zers.) (MeOH).

**Beispiel 4**: 2-(3-Methoxy-5-methylsulfinyl-2-thienyl)-1H-imidazo[4,5-b]pyridin-hydrochlorid

3,0 g (10,8 mMol) des nach Beispiel 2 erhaltenen 2-(3-Methoxy-5-methylsulfinyl-2-thienyl)-1H-imidazo[4,5-b]pyridins werden in 80 ml Methanol suspendiert und mit 14 ml ln methanolischer Salzsäure versetzt. Dann wird im Vakuum zur Trockene eingedampft und der Rückstand aus Äthanol umkristallisiert. Ausbeute 3 g (95,5 %) gelbe Kristalle, Fp. 210°C (Zers.).

Analog wird erhalten:

2-(3-Methoxy-5-methylthio-2-thienyl)-1H-imidazo[4,5-b]-pyridinhydrochlorid, Fp. 217°C (Zers.).

2-(3,5-Dimethoxy-2-thienyl)-1H-imidazo[4,5-b]pyridin-hydrochlorid, Fp. 145°C (Zers.).

Wie bereits erwähnt, besitzen die erfindungsgemäßen Verbindungen wertvolle pharmakologische Eigenschaften, insbesondere eine positiv inotrope Wirksamkeit auf das Herz, was im folgenden an Hand einer repräsentativen erfindungsgemäßen Verbindung, nämlich 2-(3-Methoxy-5-methylsulfinyl-2-thienyl)-1H-imidazo[4,5-b]pyridin-hydrochlorid (erhalten gemäß Beispiel 4) demonstriert werden soll.

Diese Verbindung wurde sowohl in vitro an isoliert perfundierten spontan schlagenden Meerschweinchen- und Rattenherzen als auch in vivo an Ratten hinsichtlich ihrer hämodynamischen Aktivität geprüft.

An den Herzen beider Spezies in vitro wirkte die Prüfsubstanz positiv inotrop, ähnlich dem Glycosid Quabain. Bei einer Dosierung von 6 x $10^{-6}$ M wurde überraschenderweise die linksventrikuläre Druckentwicklung um 130 % und die maximale Druckanstiegsgeschwindigkeit um etwa 120 % erhöht.

In Gegenwart der Testsubstanz kam es zu keiner Beeinträchtigung der myocardialen Sauerstoffversorgung.

In vivo wurden an der Ratte in Inactin®-Narkose über ein spezielles Ultraminiatur-Kathetertip-Manometer folgende hämodynamische Parameter bestimmt: Herzfrequenz, linksventrikulärer systolischer Druck (LVSP) und Anstiegsgeschwindigkeit des linksventrikulären Druckes (LV dP/dt max). (Methode beschrieben von

Zimmer, H. -G.: Basic Research Cardiol. 78, 77-84, 1983).

Der getestete Dosisbereich lag zwischen 0,1 mg/kg und 20 mg/kg/h. Die in vivo-Untersuchung bestätigte die in vitro gefundene positiv inotrope Wirkung.

In der folgenden Tabelle sind die Daten von zwei repräsentativen Experimenten zusammengestellt.

| Dosierung 20 mg/kg/h | Herzfrequenz (Schläge/min) | LV dP/dt max (mm Hg/s) | LVSP (mm Hg) |
|---|---|---|---|
| vor d. Applikation | 316 | 6.769 | 143 |
| nach d. Applikation | 440 | 10.154 | 129 |
| vor d. Applikation | 336 | 5.230 | 136 |
| nach d. Applikation | 992 | 12.600 | 136 |

Es fiel auf, daß der positiv inotrope Effekt auch nach Absetzen der Infusion noch relativ lange vorhanden war.

Zusammenfassend kann man sagen, daß es sich bei 2-(3-Methoxy-5-methylsulfinyl-2-thienyl)-1H-imidazo[4,5-b]-pyridin-hydrochlorid aus therapeutischer Sicht zur Behandlung der myocardialen Insuffizienz um eine hochinteressante Substanz handelt, die die hochtoxischen Glycoside ersetzen könnte.

**Patentansprüche**

1. 2-(2-Thienyl)-imidazo[4,5-b]pyridine der allgemeinen Formel

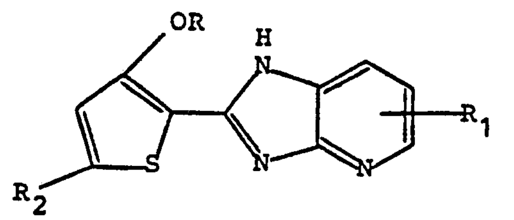

(I),

worin R Methyl oder Äthyl, $R_1$ Wasserstoff oder Methyl und $R_2$ Methylthio, Methylsulfinyl oder Methoxy bedeuten, und ihre pharmazeutisch verträglichen Säureadditionssalze.

2. 2-(3-Methoxy-5-methylthio-2-thienyl)-1H-imidazo[4,5-b]pyridin und dessen Hydrochlorid.

3. 2-(3-Methoxy-5-methylsulfinyl-2-thienyl)-1H-imidazo[4,5-b]pyridin und dessen Hydrochlorid.

4. 2-(3,5-Dimethoxy-2-thienyl)-1H-imidazo[4,5-b] pyridin und dessen Hydrochlorid.

5. Verfahren zur Herstellung von Verbindungen der Formel (I), worin R, $R_1$ und $R_2$ die in Anspruch 1 angegebene Bedeutung haben, dadurch gekennzeichnet, daß man

a) eine Verbindung der allgemeinen Formel

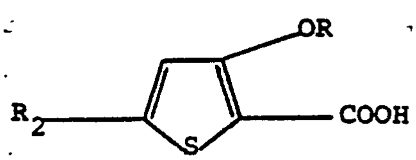

(II),

worin R die obige Bedeutung besitzt und $R_2$ Methylthio, oder Methoxy bedeutet, mit einer Verbindung der allgemeinen Formel

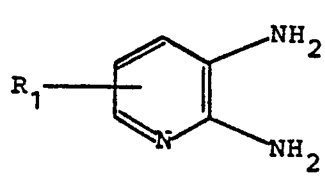

(III),

worin $R_1$ die obige Bedeutung besitzt, in Gegenwart von Phosphoroxychlorid oder Thionylchlorid umsetzt, und

b) gegebenenfalls die so erhaltene Verbindung der Formel (I), in der $R_2$ Methylthio bedeutet, mit organischen Persäuren oder Wasserstoffperoxid in eine Verbindung der allgemeinen Formel (I) umwandelt, in der $R_2$ Methylsulfinyl bedeutet, und gegebenenfalls eine erhaltene Verbindung der Formel (I) in ihr

pharmazeutisch verträgliches Säureadditionssalz überführt.

6. Verfahren nach Anspruch 5 a) zur Herstellung von 2-(3-Methoxy-5-methylthio-2-thienyl)-1H-imidazo[4,5-b]pyridin, dadurch gekennzeichnet, daß man 3-Methoxy-5-methylthio-2-thiophencarbonsäure mit 2,3-Diaminopyridin in $POCl_3$ umsetzt.

7. Verfahren nach Anspruch 5b) zur Herstellung von 2-(3-Methoxy-5-methylsulfinyl-2-thienyl)-1H-imidazo[4,5-b]pyridin, dadurch gekennzeichnet, daß man das erhaltene 2-(3-Methoxy-5-methylthio-2-thienyl)-1H-imidazo[4,5-b]pyridin mit Wasserstoffperoxid in Eisessig umsetzt.

8. Verfahren nach Anspruch 5a) zur Herstellung von 2-(3,5-Dimethoxy-2-thienyl)-1H-imidazo[4,5-b]-pyridin, dadurch gekennzeichnet, daß man 3,5-Dimethoxy-2-thiophencarbonsäure mit 2,3-Diaminopyridin in $POCl_3$ umsetzt.

9. Verfahren nach Anspruch 5 zur Herstellung von 2-(3-Methoxy-5-methylthio-2-thienyl)-1H-imidazo[4,5-b]pyridinhydrochlorid, dadurch gekennzeichnet, daß man das erhaltene 2-(3-Methoxy-5-methylthio-2-thienyl)-1H-imidazo[4,5-b]pyridin mit methanolischer Salzsäure umsetzt.

10. Verfahren nach Anspruch 5 zur Herstellung von 2-(3-Methoxy-5-methylsulfinyl-2-thienyl)-1H-imidazo[4,5-b]pyridin-hydrochlorid, dadurch gekennzeichnet, daß man das erhaltene 2-(3-Methoxy-5-methylsulfinyl-2-thienyl)-1H-imidazo-[4,5-b]pyridin mit methanolischer Salzsäure umsetzt.

11. Verfahren- nach Anspruch 5 zur Herstellung von 2-(3,5-Dimethoxy-2-thienyl)-1H-imidazo[4,5-b]pyridinhydrochlorid, dadurch gekennzeichnet, daß man das erhaltene 2-(3,5-Dimethoxy-2-thienyl)-1H-imidazo[4,5b]pyridin mit methanolischer Salzsäure umsetzt.

12. Pharmazeutische Zusammensetzung, dadurch gekennzeichnet, daß sie eine Verbindung gemäß einem der Ansprüche 1 bis 4 zusammen mit einem pharmazeutisch annehmbaren Träger enthält.

## Claims

2. 2-(2-Thienyl)-imidazo(4,5-b)pyridines corresponding to the following general formula:

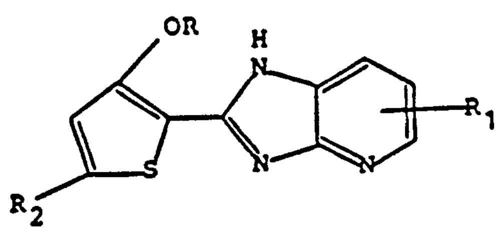

(I),

wherein R denotes methyl or ethyl, $R_1$ denotes hydrogen or methyl and $R_2$ denotes methylthio, methylsulphinyl or methoxy, and their pharmaceutically compatible acid addition salts.

2. 2-(3-Methoxy-5-methylthio-2-thienyl)-1H-imidazo(4,5-b)pyridine and its hydrochloride.

3. 2-(3-Methoxy-5-methylsulphinyl-2-thienyl)-1H-imidazo(4,5-b)pyridine and its hydrochloride.

4. 2-(3,5-Dimethoxy-2-thienyl)-1H-imidazo(4,5-b)pyridine and its hydrochloride

5. Process for the preparation of compounds corresponding to formula (I) wherein R, $R_1$ and $R_2$ have the meanings indicated in claim 1, characterised in that

a) a compound corresponding to the following general formula:

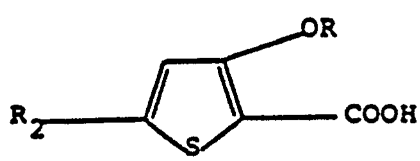

(II),

wherein R has the meaning indicated above and $R_2$ denotes methylthio or methoxy is reacted with a compound of the following general formula:

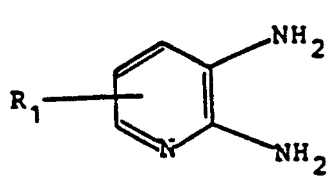

(III),

wherein $R_1$ has the meaning indicated above in the presence of phosphorus oxychloride or thionyl chloride and

b) the resulting compound of formula (I) in which $R_2$ stands for methylthio is optionally converted into a

compound corresponding to the general formula (I) in which $R_2$ stands for methylsulphinyl by reaction with organic peracids or hydrogen peroxide, and a compound of formula (I) obtained is optionally converted into a pharmaceutially compatible acid addition salt thereof.

6. Process according to claim 5a) for the preparation of 2-(3-methoxy-5-methylthio-2-thienyl)-1H-imidazo(4,5-b)pyridine, characterised in that 3-methoxy-5-methylthio-2-thiophene-carboxylic acid is reacted with 2,3-diaminopyridine in $POCl_3$.

7. Process according to claim 5b) for the preparation of 2-(3-methoxy-5-methylsulphinyl-2-thienyl)-1H-imidazo(4,5-b)pyridine, characterised in that the 2-(3-methoxy-5-methylthio-2-thienyl)-1H-imidazo(4,5-b)pyridine obtained is reacted with hydrogen peroxide in glacial acetic acid.

8. Process according to claim 5a) for the preparation of 2-(3,5-dimethoxy-2-thienyl)-1H-imidazo(4,5-b)-pyridine, characterised in that 3,5-dimethoxy-2-thiophene-carboxylic acid is reacted with 2,3-diaminopyridine in $POCl_3$.

9. Process according to claim 5 for the preparation of 2-(3-methoxy-5-methylthio-2-thienyl)-1H-imidazo(4,5-b)pyridine hydrochloride, characterised in that the 2-(3-methoxy-5-methylthio-2-thienyl)-1H-imidazo(4,5-b)pyridine obtained is reacted with methanolic hydrochloric acid.

10. Process according to claim 5 for the preparation of 2-(3-methoxy-5-methylsulphinyl-2-thienyl)-1H-imidazo(4,5-b)-pyridine hydrochloride, characterised in that the 2-(3-methoxy-5-methylsulphinyl-2-thienyl)-1H-imidazo(4,5-b)pyridine obtained is reacted with methanolic hydrochloric acid.

11. Process according to claim 5 for the preparation of 2-(3,5-dimethoxy-2-thienyl)-1H-imidazo(4,5-b)pyridine hydrochloride, characterised in that the 2-(3,5-dimethoxy-2-thienyl)-1H-imidazo(4,5-b)pyridine obtained is reacted with methanolic hydrochloric acid.

12. Pharmaceutical composition, characterised in that it contains a compound according to one of the claims 1 to 4 together with a pharmaceutically acceptable carrier.

## Revendications

1. 2-(2-thiényl)-imidazo[4,5-b]pyridines de formule générale

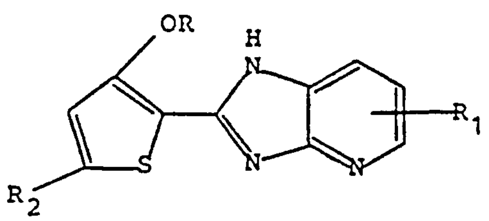

(I),

dans laquelle R est un groupe méthyle ou éthyle, $R_1$ est l'hydrogène ou un groupe méthyle et $R_2$ est un groupe méthylthio, méthylsulfinyle ou méthoxy, et leurs sels d'addition d'acides pharmaceutiquement acceptables.

2. La 2-(3-méthoxy-5-méthylthio-2-thiényl)-1H-imidazo[4,5-b]pyridine et son chlorhydrate.

3. La 2-(3-méthoxy-5-méthylsulfinyl-2-thienyl)-1H-imidazo[4,5-b]pyridine et son chlorhydrate.

4. La 2-(3,5-diméthoxy-2-thiényl)-1H-imidazo[4,5-b]pyridine et son chlorhydrate.

5. Procédé de préparation de composés de formule (I) dans laquellé R, $R_1$ et $R_2$ ont la définition indiquée dans la revendication 1, caractérisé en ce que:

a) on fait réagir un composé de formule générale

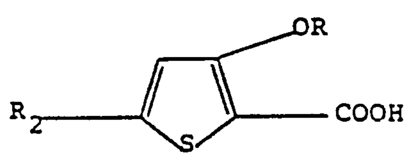

(II),

dans laquelle R a la définition indiquée ci-dessus et $R_2$ est un groupe méthylthio ou méthoxy, avec un composé de formule générale

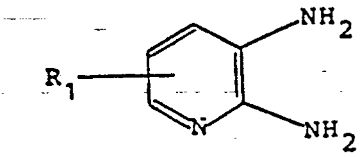

(III),

dans laquelle $R_1$ a la définition indiquée ci-dessus, en présence d'oxychlorure de phosphore ou de chlorure de thionyle, et

b) on transforme éventuellement le composé ainsi obtenu de formule (I), dans laquelle $R_2$ est un groupe méthylthio, avec des peracides organiques ou du peroxyde d'hydrogène, en un composé de formule générale (I) dans laquelle $R_2$ est un groupe méthylsulfinyle et on transforme éventuellement un composé obtenu de formule (I) en son sel d'addition d'acide pharmaceutiquement acceptable.

6. Procédé suivant la revendication 5 a) pour la préparation de 2-(méthoxy-5-méthylthio-2-thiényl)-1H-imidazo[4,5-b]pyridine, caractérisé en ce qu'on fait réagir l'acide 3-méthoxy-5-méthylthio-2-thiophènecarboxylique avec la 2,3-diaminopyridine dans du $POCl_3$.

7. Procédé suivant la revendication 5 b) pour la préparation de 2-(3-méthoxy-5-méthylsulfinyl-2-thiényl)-1H-imidazo[4,5-b]pyridine, caractérisé en ce qu'on fait réagir la 2-(3-méthoxy-5-méthylthio-2-thiényl)-1H-imidazo[4,5-b]pyridine obtenue avec le peroxyde d'hydrogène dans l'acide acétique cristallisable.

8. Procédé suivant la revendication 5 a) pour la préparation de la 2-(3,5-diméthoxy-2-thiényl)-1H-imidazo[4,5b]pyridine, caractérisé en ce qu'on fait réagir l'acide 3,5-diméthoxy-2-thiophènecarboxylique avec la 2,3-diaminopyridine dans le $POCl_3$.

9. Procédé suivant la revendication 5 pour la préparation du chlorhydrate de 2-(3-méthoxy-5-méthylthio-2-thiényl)-1H-imidazo[4,5-b]pyridine, caractérisé en ce qu'on fait réagir la 2-(3-méthoxy-5-méthylthio-2-thiényl)-1H-imidazo[4,5-b]pyridine obtenue avec l'acide chlorhydrique en solution méthanolique.

10. Procédé suivant la revendication 5 pour la préparation du chlorhydrate de 2-(3-méthoxy-5-methylsulfinyl-2-thiényl)-1H-imidazo[4,5-b]pyridine, caractérisé en ce qu'on fait réagir la 2-(3-méthoxy-5-méthylsulfinyl-2-thiényl)-1H-imidazo[4,5-b]pyridine obtenue avec l'acide chlorhydrique en solution méthanolique.

11. Procédé suivant la revendication 5 pour la préparation du chlorhydrate de 2-(3,5-diméthoxy-2-thiényl)-1H-imidazo[4,5-b]pyridine, caractérisé en ce qu'on fait réagir la 2-(3,5-diméthoxy-2-thiényl)-1H-imidazo[4,5-b]pyridine obtenue avec l'acide chlorhydrique en solution méthanolique.

12. Composition pharmaceutique, caractérisée en ce qu'elle contient un composé suivant l'une des revendications 1 à 4 en association avec un support acceptable du point de vue pharmaceutique.